# FASCICULE DE BREVET EUROPEEN

(11) **EP 2 449 044 B1**
(45) Date de publication et mention de la délivrance du brevet: **06.01.2016**
(21) Numéro de dépôt: 10734792.4
(22) Date de dépôt: 16.06.2010
(51) Int. Cl.: A61K 9/28, C09D 151/00, C09D 101/28

(54) **COMPOSITION D'ENROBAGE COMPRENANT DES POLYMERES MARQUES**
BESCHICHTUNGSZUSAMMENSETZUNG MIT MARKIERTEN POLYMEREN
COATING COMPOSITION INCLUDING MARKED POLYMERS

(30) Priorité: 29.06.2009 FR 0954429
(43) Date de publication de la demande: 09.05.2012
(73) Titulaire: Societe D'Exploitation De Produits Pour Les Industries Chimiques Seppic, 75007 Paris (FR)
(72) Inventeur: DUCCINI, Yves, F-06250 Mougins (FR); LEFEBVRE, Sandra, F-81100 Castres (FR); TROUVE, Gérard, F-81100 Castres (FR)
(74) Mandataire: Grout de Beaufort, François-Xavier
(86) Numéro de dépôt international: PCT/FR2010/051197
(87) Numéro de publication internationale: WO 2011/001058

(56) Documents cités:
- US-A- 5 776 713

## Description

La présente invention a pour objet des compositions filmogènes destinées à l'enrobage de formes solides ingérables, qu'il s'agisse de médicaments, de compléments alimentaires, de formes à visée cosmétique, de confiserie ou de sucrerie et plus particulièrement des comprimés pharmaceutiques, par exemple ceux destinés à un usage gastrique, à un usage entérique ou à un usage gastro-entérique, les procédés de préparation desdites compositions filmogènes, les procédés d'enrobage mettant en oeuvre lesdites compositions filmogènes, et les produits pharmaceutiques enrobés obtenus par la mise en oeuvre des procédés d'enrobage comprenant l'utilisation des dites compositions filmogènes.

Les fabricants de composés pharmaceutiques et notamment des comprimés ingérables sont de plus en plus confrontés au fléau de la contrefaçon de ces produits. Les produits contrefaisants sont élaborés par des fabricants n'ayant pas les autorisations des autorités compétentes, ou alors par des fabricants contrefacteurs de brevets. Il existe également des fabricants de comprimés de placebos dans lesquels on ne trouve pas de substance active. Le matériau d'un produit contrefaisant peut être le même, ou différent du matériau d'un véritable produit breveté. Souvent, le produit contrefaisant est le même, mais de qualité inférieure que l'authentique. Il est souvent difficile de distinguer deux produits chimiques ayant une même formule chimique mais élaborés par deux fabricants différents. Cela est particulièrement vrai si les deux fabricants utilisent le même processus de production Pour cette raison, il n'est pas difficile pour les contrefacteurs d'établir la formule chimique d'un ingrédient actif dans une composition, et les quantités relatives des différents ingrédients dans la composition, puis vendre son propre produit comme celui d'un autre fabricant.

Un problème à résoudre est donc de développer des moyens de lutte contre les contrefaçons de produits pharmaceutiques. Des procédés ont déjà été développés comme par exemple le marquage des emballages avec des codes barres ou des hologrammes, le marquage des formes solides avec des encres spéciales ou encore la réalisation de comprimés aux formes géométriques compliquées, difficiles à reproduire par les contrefacteurs. Aucune de ces technologies n'est totalement satisfaisante : le marquage des emballages est relativement simple à réaliser mais n'évite pas l'introduction de comprimés contrefaits à l'intérieur de boîtiers recyclés, volés ou eux même contrefaits. L'utilisation d'encres spéciales ne peut être envisagée sur tout type de support solide et demande des équipements spécifiques onéreux. Enfin la réalisation de comprimés de forme complexe est particulièrement coûteuse et techniquement compliquée. Il y a clairement un besoin d'une méthode simple pouvant être utilisée dans le domaine de la détection des tentatives de contrefaçons de produits.

La présente invention concerne alors une composition filmogène d'enrobage de comprimés pharmaceutiques comprenant des polymères, dont au moins un, noté (P), comprend au moins 50% molaire d'au moins un monomère choisi parmi le groupe constitué de l'acide acrylique, l'acide méthacrylique, l'acide itaconique ou l'acide maléique, dont une proportion massique non nulle est greffée à des marqueurs. Lesdits marqueurs étant détectables par des « kits » de détection révélateurs d'une réaction colorée entre un réactif de type anticorps déposé sur une bandelette test, par exemple, et lesdits marqueurs.

La mise en oeuvre de la composition filmogène selon l'invention est réalisée en suivant un procédé conventionnel de pelliculage de formes sèches.

Les procédés de pelliculage sont plus rapides que les procédés de dragéification, car les quantités de film déposées sont plus faibles ; elles ne représentent que 3% à 5% de la masse de substrat enrobé. Ils nécessitent la mise en oeuvre d'un équipement particulier consistant en un dispositif de pulvérisation associé à un système de séchage performant, car les quantités d'eau à évaporer sont importantes. Les procédés de pelliculage comprennent la préparation d'une dispersion, de préférence aqueuse, contenant un polymère filmogène, un système colorant, de préférence des pigments ou des laques, et éventuellement un plastifiant, des charges et/ou des additifs technologiques, puis sa pulvérisation sur les comprimés en mouvement dans une turbine rotative perforée ou dans un lit fluidisé. Un courant d'air chaud, entrant à une température supérieure ou égale à 40°C, assure le séchage de la dispersion pulvérisée et provoque la coalescence du film autour des comprimés. La couche déposée a une épaisseur de quelques dizaines de microns ; elle est opaque et colorée de manière homogène. Les polymères filmogènes les plus couramment utilisés sont des dérivés acryliques ou cellulosiques. Les polymères filmogènes acryliques sont principalement utilisés pour réaliser des formes pelliculées destinées à un usage entérique ou à un usage impliquant une libération prolongée du principe actif médicamenteux.

C'est pourquoi, la présente invention a pour objet une composition d'enrobage (C) comprenant pour 100% de sa masse :
- de 0,01% à 30% massique d'un polymère (P) comprenant au moins 50% molaire d'au moins un monomère choisi parmi le groupe constitué de l'acide acrylique, l'acide méthacrylique, l'acide itaconique ou l'acide maléique,
- de 15% à 70% massique d'au moins un agent auxiliaire d'enrobage,
- si besoin, le reste étant un agent ou un mélange d'agents choisis parmi au moins un agent plastifiant, un agent de coloration ou une charge inerte;
caractérisée en ce que des marqueurs de type haptène sont greffés sur une proportion massique non nulle dudit polymère (P), et en ce que la masse molaire moyenne en poids Mw dudit polymère (P) est comprise entre 1000 g.Mol⁻¹ et 4500 g.Mol⁻¹.

Selon un aspect particulier de l'invention, la proportion massique de polymère (P) est comprise entre 0,05% et 20% et de manière préférée entre 0,1% et 15%.

Selon un aspect particulier de l'invention, ledit polymère (P) est partiellement ou totalement salifié.

Par monomères possédant une fonction acide faible, présent dans le polymère (P) on désigne, par exemple, les monomères possédant au moins une fonction acide carboxylique, comme par exemple l'acide acrylique, l'acide méthacrylique, l'acide itaconique, l'acide maléique, l'acide fumarique, partiellement ou totalement salifiés.

Dans le polymère (P), les monomères possédant une fonction acide faible peuvent être associés à des monomères possédant une fonction acide fort et/ou à des monomères neutres.

Par monomères possédant une fonction acide fort, on désigne particulièrement les monomères possédant une fonction acide sulfonique ou une fonction acide phosphonique, partiellement ou totalement salifiée. Ledit monomère peut être par exemple l'acide styrènesulfonique partiellement ou totalement salifié; il est de préférence l'acide 2-méthyl 2-[(1-oxo 2-propènyl) amino] 1-propanesulfonique partiellement ou totalement salifiée sous forme d'un sel de métal alcalin ou de sel d'ammonium.

Par monomères neutres, on désigne particulièrement l'acrylamide, le méthacrylamide, le N-méthyl acrylamide, le N,N-diméthyl acrylamide, le diacétoneacrylamide, le N-isopropyl acrylamide, le tertio-butyl acrylamide, le N-[2-hydroxy-1,1-bis(hydroxyméthyl) éthyl] propénamide [ou tris(hydroxyméthyl) acrylamidométhane ou N-tris(hydroxyméthyl) méthyl acrylamide dénommé aussi THAM], l'acrylate de (2-hydroxy éthyle), l'acrylate de (2,3-dihydroxy propyle), le méthacrylate de (2-hydroxy éthyle), le méthacrylate de (2,3-dihydroxy propyle).

Par polymère (P) à base d'au moins un monomère possédant une fonction acide faible, on désigne par exemple :
- l'homopolymère de l'acide acrylique, l'homopolymère de l'acide méthacrylique, l'homopolymère de l'acide maléique,
- les copolymères d'acide acrylique et d'acide 2-acrylamido-2-methylpropane sulfonique, les copolymères d'acide méthacrylique et d'acide 2-acrylamido-2-methylpropane sulfonique, les copolymères d'acide maléique et d'acide 2-acrylamido-2-methylpropane sulfonique,
- les copolymères d'acide acrylique et d'acrylamide, les copolymères d'acide acrylique et du tertio-butyl acrylamide, les copolymères d'acide acrylique et de N-méthyl acrylamide, les copolymères d'acide acrylique et de N,N-diméthyl acrylamide, les copolymères d'acide acrylique et de l'acrylate de (2-hydroxy éthyle), les copolymères d'acide acrylique et du N-isopropyl acrylamide,
- les terpolymères d'acide acrylique et d'acrylamide et d'acide 2-acrylamido-2-methylpropane sulfonique, les terpolymères d'acide acrylique et de N-méthyl acrylamide et d'acide 2-acrylamido-2-methylpropane sulfonique, les terpolymères d'acide acrylique et de N,N-diméthyl acrylamide et d'acide 2-acrylamido-2-methylpropane sulfonique, les terpolymères d'acide acrylique et de tertio-butyl acrylamide et d'acide 2-acrylamido-2-methylpropane sulfonique.

Ledit polymère (P) peut, par exemple, être plus particulièrement un homopolymère d'acide acrylique, se caractérisant par une masse molaire moyenne en poids Mw = 2000 g.Mol⁻¹ et une dispersion des masses Mw/Mn < 1,3 ; Mn étant la masse molaire moyenne en nombre. Un autre exemple de polymère (P) plus particulier est un copolymère comprenant 77% molaire d'acide acrylique et 23% molaire d'acide 2-acrylamido-2-methylpropane sulfonique (AMPS), dont la masse Mw = 4500 g.Mol⁻¹.

Un autre exemple de polymère (P) plus particulier est un terpolymère comprenant 72% molaire d'acide acrylique, 18% molaire d'AMPS et de 10% molaire de tertio-butyl acrylamide, dont la masse Mw = 4500 g.Mol⁻¹.

Un autre exemple de polymère (P) plus particulier est un homopolymère d'acide maléique de masse Mw = 1000 g.Mol⁻¹.

Tous ces exemples de polymères sont préparés selon un procédé de polymérisation en phase aqueuse selon un procédé de polymérisation radicalaire impliquant un initiateur de type persulfate de sodium et un agent réducteur de type bisulfite de sodium.

Les polymères (P), tels que définis ci-dessus sont des polymères non réticulés et non branchés.

Le marqueur doit être de préférence un haptène, composé visuellement indétectable et soluble dans l'eau, susceptible de réagir avec un anticorps pour donner un composé coloré détectable à l'oeil. Il devra également pouvoir être compatible avec l'ensemble des composants de la composition d'enrobage (C) et sera non toxique. Un haptène utilisé comme marqueur est par exemple l'acide m-phenoxybenzoique. D'autres marqueurs préférés sont l'erythrosine, l'acide 4-aminonaphthalène-1-sulphonique, amarante, dafcol brun, l'acide 4-amino-1,1-azobenzène-3,4'-disulphonique, l'acide 4-hydroxy-3-methoxyphenyl-3-butèn-2-one vanilline, l'acide ethyl-4-hydroxy-3-methoxycinnamique, l'acide 7-amino-4-methylcoumarine, chromotrope FB, ponceau 4R, ponceau S, bieberich écarlate, tropaelin O curcumin, l'aclcool coniferyl, hexyl vanillate, l'acetovanillone.

De manière préférée, le marqueur utilisé dans la présente invention est le Ponceau 4R de formule (I) :

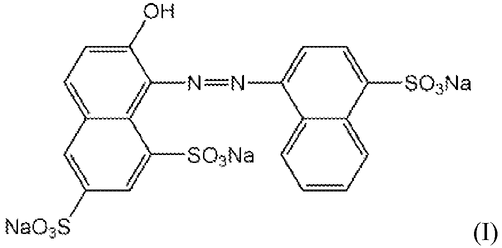

Généralement les composés pouvant être utilisés comme marqueurs sont des composés organiques comportant des atomes d'hydrogène et de carbone et un ou plusieurs hétéroatomes choisis parmi l'oxygène et l'azote. En option un ou plusieurs autres hétéroatomes peuvent également être présents, par exemple des halogènes comme le chlore, le brome ou l'iode ou encore des atomes de phosphore et de soufre. D'autres composés préférés sont les acides carboxyliques, les esters d'acides carboxyliques, cétones, alcools, phénols, amines, anilines, nitriles et les éthers, et leurs sels Plus préférentiellement ce sont des composés aromatiques acides carboxyliques, par exemple les acides benzoïques, phénols, éthers de polyalcools et des phénols, des acides aminés, peptides, protéines, lipides, glucides, par exemple, les sucres et les polysaccharides, les acides nucléiques. De préférence, seulement des traces de marqueurs sont utilisées. Généralement, un marqueur est greffé sur un polymère (P) par liaison covalente, à une concentration comprise dans la gamme de 1 partie par milliard (ppb) à 25 parties par million (ppm). De préférence la concentration sera de l'ordre de 100 ppb à 15 ppm, de préférence encore de 1ppm à 10 ppm.

La capacité à détecter des concentrations de marqueurs au dessous de 25 ppm est un avantage particulier de la méthode selon l'invention. Ainsi, seules de petites quantités de composés marqueurs doivent être utilisées.

Selon la présente invention, le taux de composés marqueurs greffés aux polymères (P) est faible. C'est-à-dire que le poids de la partie marqueur représente moins de 5% du poids dudit polymère (P) greffé avec le marqueur. Ainsi la structure polymère (P) greffé n'altère en rien l'efficacité du comprimé ou du produit enrobé par la composition d'enrobage objet de la présente invention.

Par agent auxiliaire d'enrobage, on désigne les composés ou les mélanges de composés solubles dans l'eau ou qui se dispersent dans l'eau, choisis parmi le groupe comprenant des polymères contenant des fonctions hydroxyles, amides ou esters, et des composés organiques de faible masse moléculaire. Les agents auxiliaires d'enrobage sont conformes aux réglementations en vigueur dans les domaines pharmaceutique, diététique ou alimentaire.

Les polymères contenant des fonctions hydroxyles, amides ou esters utilisés comme agents auxiliaires d'enrobage sont en général choisis parmi :
- les alcools polyvinyliques, les polysaccharides, les celluloses, les amidons, les polymères de l'acide lactique, les polyéthylène-glycols, les polypropylène-glycols ou les copolymères blocs comprenant des polyéthylène-glycols et des polypropylène-glycols, ainsi que leurs dérivés ;
- les polyvinyl pyrrolidones, les copolymères de la vinyl pyrrolidone et de l'acétate vinylique ;
- les copolymères de la vinyl pyrrolidone et de l'acide méthacrylique, les copolymères de la vinyl pyrrolidone et de l'acide acrylique ;
- les copolymères (méth)acryliques, les copolymères hydroxyalkyl (méth)acryliques comme ceux décrits dans la demande de brevet allemand publiée sous le numéro DE 1004 9297, les acétates polyvinyliques ; et
- la gélatine ;
- des polymères naturels comme la gomme-laque ou la gomme d'acacia.

Selon un aspect particulier de la présente invention, les polymères contenant des fonctions hydroxyles, amides ou esters utilisés comme agents auxiliaires d'enrobage, sont choisis parmi les alcools polyvinyliques ayant un taux d'hydrolyse compris entre 80% et 99% molaire, les hydroxypropylméthyl celluloses (HPMC), les hydroxypropyl celluloses (HPC), les hydroxyéthylméthyl celluloses, les méthyl celluloses, les carboxyméthyl celluloses de sodium, l'amylose, les maltodextrines, les sirops de glucose, les cyclodextrines, les dextranes, l'inuline, les polyfructose, les alginates tels que l'alginate de propylène glycol, les pectines, les carraghénates, le guar, les gommes xanthanes, les gommes arabiques, les terpolymères du méthacrylate de butyle et du méthacrylate de 2-(diméthylamino) éthyle et du méthacrylate de méthyle, les copolymères de l'acide méthacrylique et de l'acrylate d'éthyle, les chitosanes, les éthyl celluloses, les polyvinyl pyrrolidones réticulées, l'acétate de polyvinyle, les celluloses et plus particulièrement les cellulose microcristallines.

Le polydextrose peut également être utilisé comme agent d'enrobage, il s'agit alors d'un polysaccharide hydrosoluble obtenu par la polymérisation du glucose, ou dextrose, dont les caractéristiques et les modes de préparation sont décrits dans les brevets américains publiés sous les numéros US 3,766,165 et US 3,876,794.

Les composés organiques de faible masse moléculaire utilisés comme agents auxiliaires d'enrobage sont en général choisis parmi l'urée, les sucres, les alcools dérivés de sucres, les dérivés de sucres ou d'alcools dérivés de sucres, les silices dont la surface spécifique est supérieure ou égale à 100 m²g⁻¹. Les composés organiques de faible masse moléculaire utilisés comme agents auxiliaires d'enrobage préférés sont le lactose, le sucrose, le glucose, le mannitol, le sorbitol, l'isomalt, le xylitol.

Selon un aspect particulier, la composition d'enrobage (C) telle que définie précédemment comprend en outre pour 100% de sa masse, de 2% à 35% massique d'un agent plastifiant ou d'un mélange d'agents plastifiants.

Par agent plastifiant, on entend principalement soit les agents plastifiants comestibles solubles comme les polyols en général et, plus particulièrement le sorbitol, le mannitol, le xylitol, le glycérol, le saccharose, les polyéthylène glycols, les propylène glycols, soit les agents plastifiants comestibles peu hydrosolubles tels que ceux comprenant une chaîne aliphatique comprenant au moins 12 atomes de carbone, par exemple, l'acide stéarique, les sels de l'acide stéarique, comme le stéarate de magnésium, le stéarate d'aluminium, l'acide stéarique polyéthoxylé, les monoglycérides d'acides gras, les diglycérides d'acides gras et leurs dérivés estérifiés par l'acide acétique, l'acide tartrique ou l'acide lactique, les esters d'acides gras et de propylène glycol, les esters d'acides gras et de sorbitol, les esters d'acides gras et de sorbitan, les esters d'acides gras et de mannitol, les esters d'acides gras et de mannitan ou encore certains sucroesters, les sucroglycérides ou les esters de polyglycérol.

Selon un autre aspect particulier, la composition d'enrobage peut contenir un ou plusieurs agents hydrofugeants, susceptibles de ralentir la pénétration de la vapeur d'eau dans les comprimés. Comme agent hydrofugeant, on peut citer les acides gras ayant au moins 12 atomes de carbones et leurs dérivés, plus particulièrement l'acide stéarique et ses dérivés, les alcools polyvinyliques, les tensio actifs caractérisés par un nombre HLB inférieur à 7.

Selon un autre aspect particulier, la composition d'enrobage (C) telle que définie précédemment, comprend en outre pour 100% de sa masse de 2% à 35% massique d'un ou plusieurs agents de coloration.

Par agent de coloration utilisés dans l'invention on désigne principalement les colorants ou les pigments, comme par exemple ceux cités dans les pharmacopées européennes ou américaines, ou dans les listes d'additifs alimentaires, référencés en Europe sous les numéros E 100 à E 172, comme, par exemple, les oxydes de fer, les oxydes de titane, de zinc ou de magnésium, les colorants absorbés sur laques d'alumines, les mica-titane, ou certains colorants naturels comme le caramel, les caroténoïdes, la riboflavine, l'indigotine, la carmine ou la chlorophylle. On peut aussi avantageusement utiliser des colorants composites constitués d'un assemblage de silicates de potassium et d'aluminium (mica), de dioxyde de titane et de colorants, tels que ceux commercialisés par la société Merck sous le nom de Candurin™.

Selon un autre aspect particulier, la composition d'enrobage (C) telle que définie précédemment, comprend en outre pour 100% de sa masse de 2% à 35% massique d'une ou plusieurs charges inertes.

Les charges inertes utilisées dans l'invention sont celles qui permettent de modifier les propriétés du matériau d'enrobage et la protection dudit matériau. A titre d'exemple de charges inertes, on mentionnera les agents opacifiants comme par exemple le talc, le kaolin, l'oxyde de titane ; les agents tensioactifs mouillants et dispersants de qualité alimentaire comme par exemple les esters de sorbitan, les esters de sorbitan éthoxylés, les huiles de ricin hydrogénées éthoxylées, le lauryl sulfate de sodium, les alcools gras éthoxylés, les acides gras éthoxylés ; les agents anti-mousses et les agents réducteurs de mousses tels que les acides gras , les silicones, les dérivés de silicone ; les agents diluants comme par exemple le lactose, le sucrose, le mannitol, le xylitol, l'isomalt, le talc ; l'hydrogénophosphate de calcium ; le stéarate de magnésium ; le monostéarate de glycérol.

Selon un autre aspect particulier, la composition (C) telle que définie précédemment, comprend en outre de 1% massique à 20% massique d'un agent facilitant l'écoulement, comme par exemple une silice colloïdale finement divisée telle que celles commercialisées par la société Degussa sous l'appellation « Aerosil™ ». Les agents auxiliaires d'enrobage tels que définis ci-dessus, présentent parfois eux même, la propriété de favoriser l'écoulement des compositions d'enrobages sous forme de poudres ou de granulés et il n'est dans ce cas pas nécessaire d'en ajouter un autre.

Selon un autre aspect particulier, l'invention a pour objet une composition d'enrobage (C) telle que définie précédemment, caractérisée en ce qu'elle se présente sous une forme prête à l'emploi contenant les mélanges de ses différents constituants sous la forme d'une dispersion aqueuse, d'une poudre ou de granulés prêts à l'emploi.

Par dispersion aqueuse, on entend les dispersions réalisées dans l'eau, dans des alcools hydrosolubles comme par exemple l'éthanol ou les mélanges d'eau et d'alcools hydrosolubles comme par exemple l'éthanol.

Les compositions prêtes à l'emploi présentent plusieurs avantages :
- La manipulation, le stockage, le contrôle d'un seul et unique produit ;
- Une meilleure reproductibilité des couleurs et des performances ;
- Une mise en dispersion plus aisée.

L'invention a aussi pour objet l'utilisation de la composition d'enrobage (C), telle que définie précédemment, pour enrober des formes solides ingérables.

Par forme solide ingérable, on désigne les formes solides ingérables par l'homme ou l'animal et quelle qu'en soit leur destination, qu'il s'agisse de médicaments, de compléments alimentaires, de formes à visée cosmétique, de confiserie ou de sucrerie. L'utilisation de la composition d'enrobage (C), telle que définie précédemment, est plus particulièrement destinée aux comprimés.

L'invention a également pour objet un procédé de préparation de la composition d'enrobage (C) telle que définie ci-dessus, et se présentant sous la forme d'une poudre, comprenant les étapes suivantes :
- une étape (a) de mélange de polymères (P) sur lesquels sont greffés des marqueurs de type haptène et, si nécessaire ou si désiré un ou plusieurs agents auxiliaires d'enrobage, un ou plusieurs agents plastifiants, un ou plusieurs agents de coloration, une ou plusieurs charges inertes et/ou un ou plusieurs agents facilitant l'écoulement,
- si nécessaire, une étape (b) de broyage du mélange issue de l'étape (a), pour former ladite composition (C).

Dans le procédé tel que défini précédemment, pour la mise en oeuvre de l'étape (a), l'ensemble des composants est ajouté de manière séquencée ou simultanée. Le mélange est ensuite généralement effectué avec un dispositif de type mélangeur à barre ou mélangeur granulateur.

L'étape (b) du procédé tel que défini ci-dessus est par exemple effectuée au moyen d'un broyeur adapté, par exemple un broyeur à couteaux, de façon à obtenir une poudre finement divisée. Un tel dispositif permet d'optimiser la granulométrie finale de la composition d'enrobage (C).

L'invention a aussi pour objet un procédé de préparation d'une composition d'enrobage (C) telle que définie précédemment et se présentant sous la forme de granulés prêts à l'emploi, comprenant les étapes suivantes :
- une étape (a1) de mouillage du polymère (P) greffé à un marqueur haptène, avec éventuellement si nécessaire ou si désiré un ou plusieurs agents auxiliaires d'enrobage, un ou plusieurs agents plastifiants, un ou plusieurs agents de coloration, une ou plusieurs charges inertes et/ou un ou plusieurs agents facilitant l'écoulement, par une solution liante, afin d'obtenir une masse humide contenant de 5% à 60% d'eau,
- une étape (b1) de séchage de la masse humide obtenue à l'étape (a1), et si désiré ou si nécessaire,
- une étape (c1) de calibrage de la masse séchée obtenue à l'étape (b1) pour obtenir ladite composition (C).

Par granulés, on entend principalement des agglomérats de plusieurs dizaines à plusieurs milliers de particules de matière, initialement individualisées, pouvant être de nature identique ou différente.

Les étapes (a1) et (b1) du procédé tel que défini ci-dessus, sont notamment effectuées successivement ou concomitamment dans un mélangeur-granulateur ou dans un lit fluidisé.

L'étape (c1) du procédé tel que défini ci-dessus, est notamment effectuée dans des broyeurs ou des tamiseurs.

L'invention a aussi pour objet un procédé de préparation d'une composition d'enrobage (C) telle que définie précédemment et se présentant sous la forme de granulés prêts à l'emploi, comprenant les étapes suivantes :
- une étape (a2) d'homogénéisation du polymère (P) greffé à un marqueur haptène, avec si nécessaire ou si désiré un ou plusieurs autres agents auxiliaires d'enrobage, un ou plusieurs agents plastifiant, un ou plusieurs agents de coloration, une ou plusieurs charges inertes et/ou un ou plusieurs agents facilitant l'écoulement, dans un lit fluidisé par soufflage d'air,
- une étape (b2) de pulvérisation progressive d'une solution généralement aqueuse sur le mélange fluidisé résultant de l'étape (a2) jusqu'à former une masse humide, et
- une étape (c2) de séchage de la masse humide résultant de l'étape (b2) par soufflage d'air chaud, pour obtenir ladite composition (C).

Dans le procédé tel que défini ci-dessus l'air chaud est généralement à une température comprise entre 70°C et 100°C.

La présente invention a également pour objet une utilisation d'un polymère (P), ayant une masse molaire moyenne en poids Mw comprise entre 1000 g.Mol⁻¹ et 4500 g.Mol⁻¹, comprenant au moins 50% molaire d'au moins un monomère choisi parmi le groupe constitué de l'acide acrylique, l'acide méthacrylique, l'acide itaconique ou l'acide maléique, dont une proportion massique non nulle est greffée à des marqueurs de type haptène, pour préparer une composition d'enrobage.

La présente invention a également pour objet une utilisation de la composition d'enrobage (C) telle que définie précédemment, pour enrober des formes solides ingérables.

La présente invention a également pour objet un comprimé à usage pharmaceutique enrobé d'une composition d'enrobage telle que définie précédemment.

Un kit de détection est fourni avec de préférence une représentation de l'aspect visuel distinctif (sur la bandelette dite bandelette test) prévu pour distinguer le produit contrefaisant du véritable produit comprenant le polymère marqué selon la présente invention. Par exemple, le kit peut être fourni avec une représentation d'une marque caractéristique de la présence du marqueur greffé sur le polymère présent dans la composition d'enrobage du comprimé.

La disponibilité de moyens d'essai sous forme de kit comprenant les bandelettes test, permettant la réaction entre le marqueur et un antigène compris sur la bandelette, permet au manipulateur de vérifier l'authenticité du produit testé sans avoir recours à des installations de laboratoire.

Un avantage de la présente invention est la facilité de contrôle. En effet la composition d'enrobage objet de la présente invention permet à un particulier comme à un huissier de tester rapidement le comprimé ainsi enrobé et de détecter si le produit est contrefaisant ou non, sans forcément avoir à pratiquer et/ou à faire pratiquer des analyses plus poussées du produit présumé contrefaisant.

Un avantage de la présente invention est dû au fait que le marqueur à détecter se trouve dans la composition d'enrobage du comprimé et donc proche de la surface extérieure. En effet, lorsque le marqueur est à la surface, il se disperse plus facilement dans une solution aqueuse lors du procédé de détection, plutôt que s'il est placé dans le principe actif du comprimé, par exemple.

Les exemples suivants illustrent l'invention sans toutefois la limiter.

### Exemple 1 : préparation d'un comprimé placebo enrobé avec une composition d'enrobage selon l'invention (comprimé C1).

1 kilogramme de comprimés placebo, dont la masse particulière est de 600 mg, et constitués par un mélange de 50% massique de lactose et 50% massique de cellulose microcristalline, sont placés dans une turbine boule de pelliculage.

Les comprimés sont ensuite pulvérisés au moyen d'un dispositif de buses, par une composition d'enrobage contenant pour 100% de sa masse :
- 45 % massique d'hydroxypropylmethycellulose (HPMC) ;
- 8,5 % massique de macrogol 40 stéarate;
- 15 % massique d'agents de coloration;
- 31,4 % de cellulose microcristalline de type Vivapur;
- 0,1 % massique d'un polymère de l'acide acrylique de masse Mw = 2000 g.Mol⁻¹, greffé au Ponceau 4R (marqueur haptène), commercialisé par Rohm et Haas sous le nom de marque Optidose^{™} 1000.

La composition d'enrobage pulvérisée sur les comprimés est ensuite séchée tout en couvrant les comprimés de telle manière qu'elle représente environ 3% de la masse totale dudit comprimé C1.

La quantité théorique d'Optidose 1000 présente dans chaque comprimé enrobé C1 est donc 600 x 0,03 x 0,001 = 0,018 mg.

Des essais de détection sont exécutés par l'intermédiaire d'un kit de détection de polymère commercialisé sous le nom de marque Optidose 96 par la société Rohm and Haas.

Pour cela, un comprimé C1 enrobé comme précédemment décrit est mis en contact avec 1 gramme d'eau purifiée dans laquelle il se désagrège (ce qui donne une concentration de 18 ppm d'Optidose dans l'eau).

50 microlitres de cette solution sont alors placés dans un tube test, dans lequel on ajoute une solution tampon de façon à compléter le tube jaugé à 2 millilitres.

100 micro litres de cette dernière solution sont ensuite prélevés pour être placés dans un tube « réactif » dans lequel une bandelette test est placée pendant une durée de 10 minutes.

L'expérimentateur note ensuite l'aspect de la bandelette test ainsi introduite dans le tube réactif comprenant du comprimé C1 dissous, de la solution tampon et de l'eau.

### Exemple 2 : préparation d'un comprimé placebo enrobé avec une composition d'enrobage selon l'état de la technique (comprimé C2).

1 kilogramme de comprimés placebo, dont la masse particulière est de 600 mg, et constitués par un mélange de 50% massique de lactose et 50% massique de cellulose microcristalline, sont placés dans une turbine boule de pelliculage.

Les comprimés sont ensuite pulvérisés au moyen d'un dispositif de buses, par une composition d'enrobage contenant pour 100% de sa masse :
- 45 % massique d'hydroxypropylmethycellulose (HPMC) ;
- 8,5 % massique de macrogol 40 stéarate;
- 15 % massique de matières colorantes;
- 31,5 % de cellulose microcristalline de type Vivapur.

La composition d'enrobage pulvérisée sur les comprimés est ensuite séchée tout en couvrant les comprimés de telle manière qu'elle représente environ 3 % de la masse totale dudit comprimé C2.

Des essais de détection sont exécutés par l'intermédiaire d'un kit de détection de polymère commercialisé sous le nom de marque Optidose 96 par la société Rohm and Haas.

Pour cela, un comprimé C2 enrobé comme précédemment décrit est mis en contact avec 1 gramme d'eau purifiée dans laquelle il se désagrège.

50 microlitres de cette solution sont alors placés dans un tube test, dans lequel on ajoute une solution tampon de façon à compléter le tube jaugé à 2 millilitres.

100 microlitres de cette dernière solution sont ensuite prélevés pour être placés dans un tube « réactif » dans lequel une bandelette test est placée pendant une durée de 10 minutes.

L'expérimentateur note ensuite l'aspect de la bandelette test ainsi introduite dans le tube réactif comprenant du comprimé C2 dissous, de la solution tampon et de l'eau.

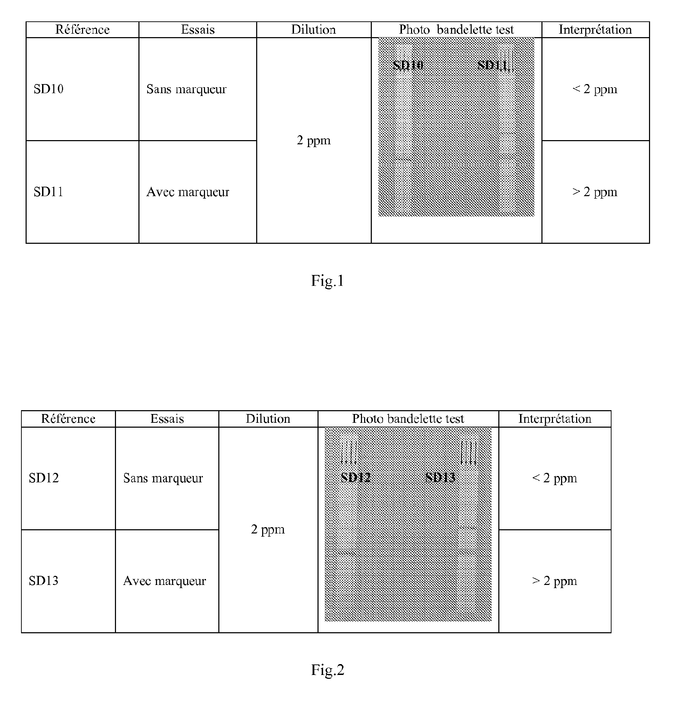

Les deux bandelettes correspondant aux tests des comprimés C1 et C2 sont comprises dans le tableau de la figure 1 qui illustre les résultats.

Sur la bandelette test de droite (référence SD11) correspondant au test du comprimé C1, enrobé avec une composition comprenant un polymère (P) marqué, il apparaît une bande de couleur supplémentaire dans la partie supérieure, indiquant une présence de marqueur supérieure à 2 ppm, que l'on ne retrouve pas sur la bandelette de gauche (référence SD10) correspondant au test du comprimé C2 enrobé avec une composition d'enrobage ne comprenant pas de polymère (P) marqué. Cette comparaison des deux bandelettes permet de conclure lequel des deux comprimés est celui qui est marqué.

### Exemple 3 : enrobage de comprimés comprenant du DICLOFENAC avec une composition d'enrobage selon l'invention.

Le DICLOFENAC est un principe actif utilisé pour traiter l'inflammation et la douleur. Il est employé dans les traitements de courte durée des rhumatismes inflammatoires et des arthroses, et pour les traitements au long cours des rhumatismes inflammatoires chroniques.

### 3.1. On prépare ici un comprimé C3 comme suit :

1 kilogramme de comprimés dosés à 25 mg par comprimé de diclofénac, dont la masse particulière est de 50 mg, et constitués par un mélange de 25 % massique de lactose et 25 % massique de cellulose microcristalline, sont placés dans une turbine boule de pelliculage.

Les comprimés sont ensuite pulvérisés au moyen d'un dispositif de buses par une composition d'enrobage contenant pour 100% de sa masse :
- de 62,5 % massique d'hydroxypropylmethycellulose (HPMC) ;
- 7,3 % massique de PEG 400;
- 30% de TiO2;
- 0,2 % massique d'un homopolymère de l'acide acrylique de masse Mw = 2000g.Mol⁻¹, greffé au Ponceau 4R (marqueur haptène), commercialisé par Rohm et Haas sous le nom de marque Optidose^{™} 1000.

La composition d'enrobage pulvérisée sur les comprimés est ensuite séchée tout en couvrant les comprimés de telle manière qu'elle représente environ 3 % de la masse totale dudit comprimé C3.

La quantité théorique d'Optidose 1000 présente dans chaque comprimé enrobé C3 est donc 50 x 0,03 x 0,002 = 0,003 mg.

Des essais de détection sont exécutés par l'intermédiaire d'un kit de détection de polymère commercialisé sous le nom de marque Optidose 96 par la société Rohm and Haas.

Pour cela, un comprimé C3 enrobé comme précédemment décrit est mis en présence d'1 gramme d'eau purifiée dans laquelle il se désagrège complètement ou partiellement. (Ce qui donne une concentration de trois (3) ppm d'Optidose dans l'eau).

50 microlitres de cette solution sont alors placés dans un tube test, dans lequel on ajoute une solution tampon pour ajuster le volume à 2 millilitres.

100 micro litres de cette dernière solution sont ensuite prélevés pour être placés dans un tube « réactif » dans lequel une bandelette test est placée pendant une durée de 10 minutes.

L'expérimentateur note ensuite l'aspect de la bandelette test ainsi introduite dans le tube réactif comprenant du comprimé C3 dissous, de la solution tampon et de l'eau.

### 3.2. On prépare ici un comprimé C4 comme suit :

1 kilogramme de comprimés dosés à 25 mg par comprimé de diclofénac, dont la masse particulière est de 50 mg, et constitués par un mélange de 25 % massique de lactose et 25 % massique de cellulose microcristalline, sont placés dans une turbine boule de pelliculage.

Les comprimés sont ensuite pulvérisés au moyen d'un dispositif de buses, par une composition d'enrobage contenant pour 100% de sa masse :
- 62,5 % massique d'hydroxypropylmethycellulose (HPMC) ;
- 7,5 % massique de PEG 400;
- 30 % de TiO2.

La composition d'enrobage pulvérisée sur les comprimés est ensuite séchée tout en couvrant les comprimés de telle manière qu'elle représente environ 3 % de la masse totale dudit comprimé C4.

Des essais de détection sont exécutés par l'intermédiaire d'un kit de détection de polymère commercialisé sous le nom de marque Optidose 96 par la société Rohm and Haas.

Pour cela, un comprimé C4 enrobé comme précédemment décrit est mis en présence d'1 gramme d'eau purifiée dans laquelle il se désagrège.

50 microlitres de cette solution sont alors placés dans un tube test, dans lequel on ajoute une solution tampon pour ajuster le volume à 2 millilitres.

100 micro litres de cette dernière solution sont ensuite prélevés pour être placés dans un tube « réactif » dans lequel une bandelette test est placée pendant une durée de 10 minutes.

L'expérimentateur note ensuite l'aspect de la bandelette test ainsi introduite dans le tube réactif comprenant du comprimé C4 dissous, de la solution tampon et de l'eau.

Les deux bandelettes correspondant aux tests des comprimés C3 et C4 sont comprises dans le tableau de la figure 2 qui illustre les résultats.

Sur la bandelette test de droite (référence SD13) correspondant au test du comprimé C3, enrobé avec une composition comprenant un polymère (P) marqué, il apparaît une bande de couleur supplémentaire dans la partie supérieure, indiquant une présence de marqueur supérieure à 2 ppm, que l'on ne retrouve pas sur la bandelette de gauche (référence SD12) correspondant au test du comprimé C4 enrobé avec une composition d'enrobage ne comprenant pas de polymère (P) marqué. Cette comparaison des deux bandelettes permet de conclure lequel des deux comprimés est celui qui est marqué.

Cette expérience est effectuée pour montrer qu'un principe actif présent dans le comprimé ainsi enrobé n'altère en rien la méthode de détection objet de la présente invention.

## Revendications

1. Composition d'enrobage (C) comprenant pour 100% de sa masse :
- de 0,01% à 30 % massique d'un polymère (P) comprenant au moins 50% molaire d'au moins un monomère choisi parmi le groupe constitué de l'acide acrylique, l'acide méthacrylique, l'acide itaconique ou l'acide maléique,
- de 15% à70 % massique d'au moins un agent auxiliaire d'enrobage,
- si besoin, le reste est un agent ou un mélange d'agents choisis parmi au moins un agent plastifiant, un agent de coloration, un agent facilitant l'écoulement ou une charge inerte;
**caractérisée en ce que** des marqueurs de type haptène sont greffés sur une proportion massique non nulle dudit polymère (P), et **en ce que** la masse molaire moyenne en poids Mw dudit polymère (P) est comprise entre 1000 g.Mol⁻¹ et 4500 g.Mol⁻¹.

2. Composition d'enrobage (C) telle que définie à la revendication 1, **caractérisée en ce que** le polymère (P) est un homopolymère d'acide acrylique de masse Mw égale à environ 2000 g.Mol⁻¹ et dont la dispersion Mw/Mn est inférieure à 1,3.

3. Composition d'enrobage (C) telle que définie à l'une des revendications 1 ou 2, **caractérisée en ce que** le marqueur est le ponceau 4R.

4. Composition d'enrobage (C) telle que définie à l'une des revendications 1 à 3, **caractérisée en ce que** ledit agent auxiliaire d'enrobage est une hydroxypropylméthyl cellulose (HPMC).

5. Composition d'enrobage (C) telle que définie à l'une des revendications 1 à 4, **caractérisée en ce qu'**elle comprend en outre de 2% à 35% massique d'un agent plastifiant ou d'un mélange d'agents plastifiants.

6. Composition d'enrobage (C) telle que définie à l'une des revendications 1 à 5, **caractérisée en ce qu'**elle comprend en outre de 2% à 35% massique d'un ou plusieurs agents de coloration.

7. Composition d'enrobage (C) telle que définie à l'une des revendications 1 à 6, **caractérisée en ce qu'**elle comprend en outre de 2% à 35% massique d'une ou plusieurs charges inertes.

8. Composition d'enrobage (C) telle que définie à l'une des revendications 1 à 7, **caractérisée en ce qu'**elle comprend en outre de 1% à 20% massique d'un agent facilitant l'écoulement.

9. Composition d'enrobage (C) telle que définie à l'une des revendications 1 à 8, **caractérisée en ce qu'**elle se présente sous la forme d'une dispersion aqueuse, d'une poudre ou de granulés prêts à l'emploi.

10. Procédé de préparation de la composition d'enrobage (C) telle que définie à l'une des revendications 1 à 9, et se présentant sous la forme d'une poudre, comprenant les étapes suivantes :
une étape (a) de mélange de polymères (P) sur lesquels sont greffés des marqueurs de type haptène et, si nécessaire ou si désiré un ou plusieurs agents auxiliaires d'enrobage, un ou plusieurs agents plastifiants, un ou plusieurs agents de coloration, une ou plusieurs charges inertes et/ou un ou plusieurs agents facilitant l'écoulement,
si nécessaire, une étape (b) de broyage du mélange issue de l'étape (a), pour former ladite composition (C).

11. Utilisation d'un polymère (P), ayant une masse molaire moyenne en poids Mw comprise entre 1000 g.Mol⁻¹ et 4500 g.Mol⁻¹, comprenant au moins 50% molaire d'au moins un monomère choisi parmi le groupe constitué de l'acide acrylique, l'acide méthacrylique, l'acide itaconique ou l'acide maléique, dont une proportion massique non nulle est greffée à des marqueurs de type haptène, pour préparer une composition d'enrobage.

12. Utilisation de la composition d'enrobage (C) telle que définie aux revendications 1 à 9, pour enrober des formes solides ingérables.

13. Comprimé à usage pharmaceutique enrobé d'une composition d'enrobage telle que définie à l'une des revendications 1 à 9.

## Patentansprüche

1. Beschichtungszusammensetzung (C), umfassend für 100 % ihrer Masse
- von 0,01 bis 30 Massenprozent eines Polymers (P), das mindestens 50 Molprozent mindestens eines Monomers umfasst, ausgewählt aus der Gruppe, bestehend aus Acrylsäure, Methacrylsäure, Itaconsäure oder Maleinsäure,
- von 15 bis 70 Massenprozent mindestens eines Hilfsbeschichtungsmittels,
- falls erforderlich, ist der Rest ein Mittel oder eine Mischung aus Mitteln, ausgewählt aus mindestens einem Weichmacher, einem Farbstoff, einem Mittel, das den Fluss erleichtert oder eines inerten Füllstoffs; **dadurch gekennzeichnet, dass** Marker vom Typ Hapten auf einen Massenanteil, der nicht gleich null ist, des Polymers (P) transplantiert werden, und dadurch, dass die mittlere Molmasse in Gewicht MW des Polymers (P) zwischen 1000 g.MoL⁻¹ und 4500 g.MoL⁻¹ liegt.

2. Beschichtungszusammensetzung (C), wie in Anspruch 1 definiert, **dadurch gekennzeichnet, dass** das Polymer (P) ein Acrylsäurehomopolymer mit Masse Mw gleich ungefähr 2000 g.MoL⁻¹ ist, und wobei die Dispersion Mw/Mn kleiner als 1,3 ist.

3. Beschichtungszusammensetzung (C), wie in einem der Ansprüche 1 oder 2 definiert, **dadurch gekennzeichnet, dass** der Marker Ponceau 4R ist.

4. Beschichtungszusammensetzung (C), wie in einem der Ansprüche 1 bis 3 definiert, **dadurch gekennzeichnet, dass** das Hilfsbeschichtungsmittel eine Hydroxypropylmethylcellulose (HPMC) ist.

5. Beschichtungszusammensetzung (C), wie in einem der Ansprüche 1 bis 4 definiert, **dadurch gekennzeichnet, dass** sie außerdem 2 bis 35 Massenprozent eines Weichmachers oder einer Mischung aus Weichmachern umfasst.

6. Beschichtungszusammensetzung (C), wie in einem der Ansprüche 1 bis 5 definiert, **dadurch gekennzeichnet, dass** sie außerdem 2 bis 35 Massenprozent eines oder mehrerer Farbstoffe umfasst.

7. Beschichtungszusammensetzung (C), wie in einem der Ansprüche 1 bis 6 definiert, **dadurch gekennzeichnet, dass** sie außerdem 2 bis 35 Massenprozent eines oder mehrere inerter Füllstoffe umfasst.

8. Beschichtungszusammensetzung (C), wie in einem der Ansprüche 1 bis 7 definiert, **dadurch gekennzeichnet, dass** sie außerdem von 1 bis 20 Massenprozent eines Mittels umfasst, das den Fluss erleichtert.

9. Beschichtungszusammensetzung (C), wie in einem der Ansprüche 1 bis 8 definiert, **dadurch gekennzeichnet, dass** sie die Form einer wässrigen Dispersion, eines Pulvers oder von Körnchen aufweist, die fertig zum Gebrauch sind.

10. Verfahren zur Herstellung der Beschichtungszusammensetzung (C), wie in einem der Ansprüche 1 bis 9 definiert, und die die Form eines Pulvers aufweist, umfassend die folgenden Schritte:
einen Schritt (a) des Mischens von Polymeren (P), auf denen Marker vom Typ Hapten implantiert werden, und, falls erforderlich oder gewünscht, ein oder mehrer Hilfsbeschichtungsmittel, ein oder mehrere Weichmacher ein oder mehrere Farbstoffe, ein oder mehrere inerte Füllstoffe und/oder ein oder mehrere Mittel, die den Fluss erleichtern,
falls erforderlich einen Schritt (b) des Mahlens der Mischung, die sich aus dem Schritt (a) ergibt, um die Zusammensetzung (C) zu bilden.

11. Verwendung eines Polymers (P) mit einer mittleren Molmasse in Gewicht Mw zwischen 1000 g.Mol⁻¹ und 4500 g.Mol⁻¹, umfassend mindestens 50 Molprozent mindestens eines Monomers, ausgewählt aus der Gruppe bestehend aus Acrylsäure, Methacrylsäure, Itaconsäure oder Maleinsäure, wobei ein Massenanteil, der nicht gleich null ist, auf Marker vom Typ Hapten implantiert wird, um eine Beschichtungszusammensetzung herzustellen.

12. Verwendung der Beschichtungszusammensetzung (C), wie in den Ansprüchen 1 bis 9 definiert, um feste einnehmbare Formen zu beschichten.

13. Tablette zur pharmazeutischen Verwendung, beschichtet mit einer Beschichtungszusammensetzung wie in einem der Ansprüche 1 bis 9 definiert

## Claims

1. Coating composition (C) comprising for 100% of its mass:
- 0.01% to 30% by mass of a polymer (P) comprising at least 50% molar of at least one monomer selected from a group consisting of acrylic acid, methacrylic acid, itaconic acid or maleic acid,
- 15% to 70% by mass of at least one auxiliary coating agent,
- if necessary, the remainder is an agent or mixture of agents selected from at least one plasticising agent, a colouring agent, a flow facilitating agent or an inert filler,
**characterised in that** the hapten type markers are grafted onto a mass proportion not equal to zero of said polymer (P), and **in that** the average molar mass by weight Mw of said polymer (P) is between 1000 g.Mol⁻¹ and 4500 g.Mol⁻¹.

2. Coating composition (C) as defined in claim 1, **characterised in that** the polymer (P) is a homopolymer of acrylic acid of mass Mw equal to about 2000 g.Mol⁻¹ and in which the dispersion of Mw/Mn is less than 1.3.

3. Coating composition (C) as defined in either claim 1 or 2, **characterised in that** the marker is Ponceau 4R.

4. Coating composition (C) as defined in any one of claims 1 to 3, **characterised in that** said auxiliary coating agent is a hydroxypropylmethyl cellulose (HPMC).

5. Coating composition (C) as defined in any one of claims 1 to 4, **characterised in that** it also comprises 2% to 35% by mass of a plasticising agent or a mixture of plasticising agents.

6. Coating composition (C) as defined in any one of claims 1 to 5, **characterised in that** it also comprises 2% to 35% by mass of one or more colouring agents.

7. Coating composition (C) as defined in any one of claims 1 to 6, **characterised in that** it also comprises 2% to 35% by mass of one or more inert fillers.

8. Coating composition (C) as defined in any one of claims 1 to 7, **characterised in that** it also comprises 1% to 20% by mass of a flow facilitating agent.

9. Coating composition (C) as defined in any one of claims 1 to 8, **characterised in that** it is in the form of an aqueous dispersion, a powder or ready-to-use granules.

10. Method for the preparation of the coating composition (C) as defined in any one of claims 1 to 9, and in the form of a powder, comprising the following steps:
a step (a) of mixing polymers (P) onto which hapten type markers are grafted and, if necessary or if desired, one or more auxiliary coating agents, one or more plasticising agents, one or more colouring agents, one or more inert fillers and/or one or more flow facilitating agents,
if necessary, a step (b) of crushing the mixture from step (a) to form said composition (C).

11. Use of a polymer (P) having an average molar mass by weight Mw of between 1000 g.Mol⁻¹ and 4500 g.Mol⁻¹, comprising at least 50% molar of at least one monomer selected from a group consisting of acrylic acid, methacrylic acid, itaconic acid or maleic acid, of which a mass proportion not equal to zero is grafted onto hapten type markers, for preparing a coating composition.

12. Use of the coating composition (C) as defined in claims 1 to 9 for coating solid unmanageable forms.

13. Tablet for pharmaceutical use coated with a coating composition as defined in any one of claims 1 to 9.
